# EUROPEAN PATENT SPECIFICATION

(11) **EP 2 608 784 B1**
(45) Date of publication and mention of the grant of the patent: **19.09.2018**
(21) Application number: 11751752.4
(22) Date of filing: 22.08.2011
(51) Int. Cl.: A61K 31/221, A61K 31/41, A61P 9/00, A61P 9/04, A61P 9/12, A61K 45/06

(54) **TREATMENT OR PREVENTION OF HEART FAILURE IN A MAMMAL RECEIVING ANTI-COAGULANT THERAPY**
BEHANDLUNG UND/ODER PRÄVENTION VON HERZINSUFFIZIENZ BEIM MENSCHEN MIT EINER ANTIKOAGULATIONSTHERAPIE
TRAITEMENT ET/OU PRÉVENTION DE L'INSUFFISANCE CARDIAQUE CHEZ UN HUMAIN RECEVANT UNE THÉRAPIE ANTICOAGULANTE

(30) Priority: 24.08.2010 US 376417 P
(43) Date of publication of application: 03.07.2013
(73) Proprietor: Novartis AG, 4056 Basel (CH)
(72) Inventor: ALBRECHT, Diego, CH-4002 Basel (CH); CHANDRA, Priyamvada, East Hanover New Jersey 07936-1080 (US); GODTFREDSEN, Sven, East Hanover New Jersey 07936-1080 (US); JORDAAN, Pierre, CH-Basel 4002 (CH); LEFKOWITZ, Martin, East Hanover New Jersey 07936-1080 (US)
(74) Representative: Larbig, Karen Dorothee
(86) International application number: PCT/US2011/048542
(87) International publication number: WO 2012/027237

(56) References cited:
- WO-A1-2007/056546
- US-A1- 2003 144 215
- RUILOPE L M ET AL: "Blood-pressure reduction with LCZ696, a novel dual-acting inhibitor of the angiotensin II receptor and neprilysin: a randomised, double-blind, placebo-controlled, active comparator study", THE LANCET, LANCET LIMITED. LONDON, GB, vol. 375, no. 9722, 10 April 2010 (2010-04-10), pages 1255-1266, XP027490781, ISSN: 0140-6736, DOI: 10.1016/S0140-6736(09)61966-8 [retrieved on 2010-04-10]
- Novartis: "Investigational Novartis drug LCZ696, a first-in-class ARNI, showspromising results in Phase II study of hypertensive patients", , 16 March 2010 (2010-03-16), XP002663549, Retrieved from the Internet: URL:http://www.theheart.org/documents/site structure/en/content/press-center/PDF/Inve stigational%20Novartis%20drug%20LCZ696,%20 a%20first-in-class%20ARNI,%20shows%20promi sing%20results%20in%20Phase%20II%20study%2 0of%20hypertensive%20patients.pdf [retrieved on 2011-11-11]
- "Safety/Tolerability, Pharmacokinetics/Pharmacodynamics (PK/PD) of LCZ696 in Patients With Stable Heart Failure", Clinical Trials.gov , 21 December 2009 (2009-12-21), XP002663550, Retrieved from the Internet: URL:http://www.clinicaltrials.gov/ct2/show /NCT00913653?term=LCZ696&rank=3 [retrieved on 2011-11-11]
- WEINBERG A D ET AL: "Quality Improvement Case Study: Warfarin Sodium Interactions", JOURNAL OF THE AMERICAN MEDICAL DIRECTORS ASSOCIATION, ELSEVIER, NL, vol. 7, no. 5, 1 June 2006 (2006-06-01), pages 315-318, XP025159325, ISSN: 1525-8610, DOI: 10.1016/J.JAMDA.2006.04.004 [retrieved on 2006-06-01]
- FANG M C ET AL: "Language, literacy, and characterization of stroke among patients taking warfarin for stroke prevention: Implications for health communication", PATIENT EDUCATION AND COUNSELING, ELSEVIER, vol. 75, no. 3, 1 June 2009 (2009-06-01), pages 403-410, XP026161579, ISSN: 0738-3991, DOI: 10.1016/J.PEC.2008.12.009 [retrieved on 2009-01-25]
- WILLIAMS SCOTT ET AL: "Cardiovascular medications.", PSYCHOSOMATICS 2007 NOV-DEC LNKD- PUBMED:18071104, vol. 48, no. 6, November 2007 (2007-11), pages 537-547, XP002663551, ISSN: 0033-3182

## Description

### Background of the Invention

As the population lives longer which results in an increased prevalence of cardiovascular risk factors and disease, and as survival following acute myocardial infarction (MI) increases, the numbers of patients living with congestive heart failure (CHF) is expanding. In parallel, a concomitant increase in the number of hospitalizations for acute decompensated heart failure (ADHF) has occurred. In the United States alone, heart failure (HF) affects 5.7 million Americans, with over 650,000 new cases diagnosed annually, with increasing hospitalization rates.

Heart failure remains as a high unmet medical need with an annual mortality rate of about 20%. Reductions in mortality and cardiovascular morbidity has been achieved by RAAS blockers (ACE inhibitors and ARBs) and beta (β)-blockers in HF. However, the therapeutic benefit of RAAS blockade with ACE inhibitors and/or ARBs are limited, possibly caused by (a) angiotensin II escape due to incomplete ACE inhibition or angiotensin II originating from alternative non-ACE pathways, and (b) other neurohormonal and other mechanisms contributing to cardiac disease and outcomes.

The compounds and pharmaceutical compositions disclosed herein include novel drug candidates useful for the treatment of hypertension and/or heart failure. Such compounds or pharmaceutical compositions have been previously disclosed in WO2007/056546, WO 2009/061713, U.S. Patent Application Publication No. 20090156585 & U.S. Patent No. 7,468,390.

Patients suffering from heart failure frequently also receive anti-coagulant therapy. Warfarin is an anti-coagulant with a narrow therapeutic window. Warfarin is known to cause hemorrhage and necrosis of skin and other tissues. Adverse reactions reported infrequently include: hypersensitivity/allergic reactions, including anaphylactic reactions, systemic cholesterol microembolization, purple toes syndrome, dermatitis, including bullous eruptions, pruritus, rash, urticaria, edema, hepatitis, cholestatic hepatic injury, jaundice, elevated liver enzymes, hypotension, vasculitis, anemia, pallor, fever, angina syndrome, chest pain, abdominal pain including cramping, flatulence/bloating, nausea, vomiting, diarrhea, fatigue, lethargy, malaise, asthenia, pain, headache, dizziness, loss of consciousness, syncope, coma, taste perversion, alopecia, cold intolerance, and paresthesia including feeling cold and chills.

There is a high potential of drug interaction of warfarin with other drugs. Hence warfarin co-administration is often contraindicated or dose adjustment may be required in patients receiving warfarin or other anticoagulant therapy given the risk associated with altered warfarin pharmacokinetic and pharmacodynamic profiles resulting in either excess bleeding or reduced anticoagulant activity.

Situations in patients receiving anticoagulant therapy which require careful monitoring include hemorrhage and necrosis, or the presence of any predisposing condition where added risk of hemorrhage, necrosis, and/or gangrene is present, heparin-induced thrombocytopenia, deep venous thrombosis, diffuse intravascular coagulation (DIC), hypercoagulability, lactation, moderate to severe hepatic or renal insufficiency, infectious disease, disturbance of intestinal flora, trauma which may cause internal bleeding, surgery, moderate to severe hypertension, deficiency in protein C mediated anticoagulant response and miscellaneous conditions like polycythemia vera, vasculitis, and severe diabetes.

Strict monitoring of warfarin use is indicated in these situations to prevent adverse outcomes. There remains a need for pharmaceutical compositions for use in treating hypertension and/or preventing or treating heart failure in persons receiving anti-coagulant treatment that do not impact warfarin treatment to avoid adverse events in subjects receiving anticoagulant treatment with warfarin.

The article RUILOPE et al, 2010 (Lancet, 375(9722),p 1255-1266) as well as an associated press release from Novartis ("Investigational Novartis drug LCZ696, a first-in-class ARNI, shows promising results in Phase II study of hypertensive patients", 16 March 2010) describe the results of a Phase 2 trial investigating the potential of blood-pressure reduction with LCZ696, a novel dual-acting inhibitor comprising an angiotensin II receptor and a neprilysin inhibitor. The two documents describe the compound LCZ696 as potent blood pressure-lowering agent in hypertensive patients, and the press release states that the drug is undergoing clinical trials for the treatment of heart failure. However, none of the documents clearly indicates if the tested patients might receive any anti-coagulant treatment.

The article WILLIAMS SCOTT et al, 2007 (Psychosomatics, 48(6), p 537-547) indicated that in the control of cardiovascular risk factors, as in the present case, polypharmacy has also become the norm, leaving space for potential drug-drug interactions. It particular discloses that the most commonly prescribed anti- coagulant drug, warfarin, is further known to raise bleeding risks and possible interactions with other drugs so that patients under warfarin treatment require close monitoring. And that angiotensin receptor-blockers such as valsartan have a low potential for drug-drug interactions compared with other classes of agents (p 540, column 2, first paragraph), which render them the treatment of choice for patients suffering from hypertension and/or heart failure and being simultaneously on warfarin therapy - a drug for which drug-drug interactions have to be closely monitored.

Whilst it is known that valsartan has a low potential for drug-drug interactions in general, and its potential interaction with warfarin had already been investigated and included in the valsartan drug label, the potential drug-drug interaction for the compound or composition in the present invention comprising the Neprilysin inhibitor N-(3-carboxy-1-oxopropyl)-(4S)-(p-phenylphenylmethyl)-4-amino-2R-methylbutanoic acid ethyl ester (also known as AHU377 or under the INN sacubitril) have never been investigated.

### Summary of the Invention

The present invention is directed towards a pharmaceutical composition for use in the prevention or treatment of heart failure in a human receiving anti-coagulant therapy comprising
a therapeutically effective amount of the compound trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate (compound of formula (I))
in combination with one or more pharmaceutically acceptable carriers,
wherein the anticoagulant treatment comprises administration of warfarin or a pharmaceutically acceptable salt thereof,
wherein the co-administration of the pharmaceutical composition and warfarin did not show pharmacokinetic drug-drug interaction.

Heart failure which can be treated by the present invention include congestive heart failure, left heart failure, right heart failure, chronic heart failure, advanced heart failure, acute heart failure, acute decompensated heart failure, heart failure with reduced ejection fraction, heart failure with preserved ejection fraction, and heart failure primarily or secondarily associated with pulmonary hypertension,

The present invention provides that the compound of formula (I) is effective to induce at least one physiological event in the human subject including vasodilation, diuresis, natriuresis and combinations thereof.

The present invention provides that therapeutically effective amount of compound of formula (I) is effective to inhibit one or more physiological mechanisms in the human subject including vasoconstriction, remodulation, hypertrophy, hyperproliferation, edema, and combinations thereof.

The present invention can include humans who have previously suffered a myocardial infarction or have an enlarged heart. The present invention can include human having or suffering from atherosclerosis or hypertension.

### Brief Description of Drawings

Figure 1a: Drug-Drug Interaction (DDI) study showing arithmetic mean steady-state concentration-time profiles (+/-standard deviations SD) of R-warfarin in the presence and absence of LCZ696 (Compound L). Figure 1a shows the lack of pharmacokinetic interaction between R-warfarin and LCZ696.
Figure 1b: DDI study showing arithmetic mean steady-state concentration-time profiles (+/-standard deviation) of S-Warfarin in the presence and absence of LCZ696. Figure 1b shows the lack of pharmacokinetic interaction between S-warfarin and LCZ696.
Figure 2: Mean with standard deviation prothrombin time (PT) in seconds (secs) following warfarin (racemic) administration in the presence and the absence (placebo) of LCZ696. Figure 2 shows the lack of interaction between warfarin and LCZ696 as indicated by prothrombin times.
Figure 3: Mean with standard deviation (SD) International Normalized Ratio (INR) following warfarin (racemic) administration. Figure 3 shows the lack of interaction between warfarin and LCZ696 as indicated by the INR.

### Detailed Description of the Invention

The present invention is based upon the surprising and unexpected discovery that certain drugs (i.e. LCZ696) effective for the treatment of cardiovascular disease or conditions, such as heart failure or hypertension, in human subjects receiving anti-coagulant therapy with warfarin, do not impact either the pharmacokinetic (PK) or pharmacodynamic (PD) profile of the anticoagulant drug (i.e. warfarin). Thus, the invention encompasses a pharmaceutical composition for use in the prevention or treatment of heart failure in a human receiving warfarin or other anti-coagulant therapy by administering a therapeutically effective amount of the compounds or pharmaceutical compositions described herein without the need to monitor and/or adjust the warfarin dosage.

Types of heart failure which can be treated by the composition for use according to the invention include, but are not limited to, acute heart failure, acute decompensated heart failure, chronic heart failure, left heart failure, right heart failure, chronic decompensated heart failure, congestive heart failure, and primary or secondary heart failure. Types of heart failure which can be treated by the composition for use according to the invention also include heart failure with reduced ejection fraction (systolic heart failure) and heart failure with preserved ejection fraction (diastolic heart failure).

In some of the embodiments of the invention, the composition for use for treating heart failure in human subjects receiving anticoagulant therapy using the compounds described herein may result from induction of vasodilation, diuresis and/or natriuresis and/or inhibition of vasoconstriction, hypertrophy, hyperproliferation and edema.

Coagulation refers to the process of liquid blood forming a solid mass, also called a thrombus.

Warfarin is an anticoagulant drug, also known as (*RS*)-4-hydroxy-3-(3-oxo-1-phenylbutyl)-2*H*-chromen-2-one. Warfarin consists of a racemic mixture of two active enantiomers, *R*- and *S*- warfarin, each of which is cleared by different pathways. Warfarin is a synthetic derivative of dicoumarol, a 4-hydroxycoumarin-derived mycotoxin anticoagulant found in spoiled clover-based animal feeds. Dicoumarol, in turn, is derived from coumarin, a chemical found naturally in numerous plants. Warfarin is often prescribed to people at an increased risk for thrombosis or as primary or secondary prophylaxis (prevention of episodes) in those individuals that have or have not formed a blood clot (thrombus). Warfarin treatment can help prevent formation of future blood clots and reduce the risk of embolism, the migration of a thrombus that could impede blood supply to an organ. Warfarin is typically administered orally as fractions or multiples of 5 mg tablets.
The prothrombin time (PT) and its derived measures of prothrombin ratio (PR) and international normalized ratio (INR) are measures of the extrinsic pathway of coagulation. They are used to determine the bleeding or clotting tendency of blood, to determine warfarin dosage, and in liver damage and other conditions that may affect vitamin K status. The prothrombin time is the time it takes blood plasma to clot after addition of tissue factor (obtained from animals). The result (in seconds) of the prothrombin time performed in a normal individual will vary depending the analytical system and method used. This is due to differences between different batches of manufacturer's tissue factor used to perform the test.
The international normalized ratio (INR) was devised to standardize the results. The INR is the ratio of a patient's prothrombin time (PT) to a normal (control) sample, raised to the power of the International Sensitivity Index (ISI) value for the analytical system used.
The invention encompasses a composition for use of treating heart failure in a human subject receiving warfarin as described herein comprising administering a therapeutically effective amount of trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate (Compound L also known as LCZ696). Such compounds and pharmaceutical compositions have been previously disclosed in WO2007/056546, WO 2009/061713.

Preferably, compound (I) or L, is substantially pure or in a substantially pure form. As used herein, "substantially pure" refers to at least about 90% purity, more preferably at least about 95% and most preferably at least about 98% purity.

Also preferred is that compound (I) or L, is solid or a solid form or solid state. The solid, solid form or solid state can be crystalline, partially crystalline, amorphous or polyamorphous, preferably in the crystalline form.

A therapeutically effective amount of the compound of the above pharmaceutical composition for use according to the present invention may be administered simultaneously or sequentially and in any order. The dosage and/or ratio of the active compound or compounds in the pharmaceutical composition may vary depending on a variety of factors, such as mode of administration, homeothermic species, age and/or individual condition. Dosages of compound (I) in the pharmaceutical composition can include but are not limited to 5 mg, 20 mg, 25 mg, 40 mg, 50mg, 80 mg, 100 mg, 200 mg, 400 mg, 800 mg and 1000 mg. Such dosages for compound (I) can be considered therapeutically effective amounts or dosage strengths. The projected efficacy in animal disease models ranges from about 0.1 mg/kg/day to about 1000 mg/kg/day given orally, and the projected dose for human treatment ranges from about 0.1 mg/day to about 2000 mg/day. Preferred ranges are from about 40 mg/day to about 960 mg/day of the linked prodrug, preferably about 40 mg/day to about 640 mg/day. In the case of oral administration, an approximate daily dose of from about 1 mg to about 360 mg is to be estimated, e.g., for a patient of approximately 75 kg in weight. Pharmaceutical compositions containing a compound of formula(I) (such as compound L), can be administered any number of times per day, i.e. once a day (q.d.), twice (b.i.d.), three times, four time, etc. in an immediate release formation or less frequently as an extended or sustained release formation. Preferably the pharmaceutical composition is administered twice daily (b.i.d.). Corresponding doses may be taken, for example, in the morning, at mid-day or in the evening.
The pharmaceutical compositions for use according to the invention can be prepared in a manner known per se and are those suitable for enteral, such as oral or rectal, and parenteral administration to humans, comprising a therapeutically effective amount of the pharmacologically active compound, alone or in combination with one or more pharmaceutically acceptable carriers, especially suitable for enteral or parenteral application. Typical oral formulations include tablets, capsules, syrups, elixirs and suspensions. Typical injectable formulations include solutions and suspensions.

The typical pharmaceutically acceptable carriers for use in the formulations described above are exemplified by sugars, such as lactose, sucrose, mannitol and sorbitol; starches, such as cornstarch, tapioca starch and potato starch; cellulose and derivatives, such as sodium carboxymethyl cellulose, ethyl cellulose and methyl cellulose; calcium phosphates, such as dicalcium phosphate and tricalcium phosphate; sodium sulfate; calcium sulfate; polyvinylpyrrolidone; polyvinyl alcohol; stearic acid; alkaline earth metal stearates, such as magnesium stearate and calcium stearate; stearic acid; vegetable oils, such as peanut oil, cottonseed oil, sesame oil, olive oil and corn oil; non-ionic, cationic and anionic surfactants; ethylene glycol polymers; betacyclodextrin; fatty alcohols; and hydrolyzed cereal solids, as well as other non-toxic compatible fillers, binders, disintegrants, buffers, preservatives, antioxidants, lubricants, flavoring agents and the like commonly used in pharmaceutical formulations.

These pharmaceutical preparations are for enteral, such as oral, and also rectal or parenteral, administration to human subjects, with the preparations comprising the pharmacological active compound either alone or together with customary pharmaceutical auxiliary substances. For example, the pharmaceutical preparations consist of between 0.1-90%, preferably between 1% to about 80%, of the active compounds. Pharmaceutical preparations for enteral or parenteral administration are, e.g., in unit dose forms, such as coated tablets, tablets, capsules, suppositories or ampoules. These are prepared in a manner which is known per se, e.g., using conventional mixing, granulation, coating, solubilizing or lyophilizing processes. Thus, pharmaceutical preparations for oral use can be obtained by combining the active compounds with solid excipients, if desired granulating a mixture which has been obtained, and, if required or necessary, processing the mixture or granulate into tablets or coated tablet cores after having added suitable auxiliary substances. Preferably the compound of formula (I) are provided in a single pill, capsule or tablet.

Without further description, it is believed that one of ordinary skill in the art can, using the preceding description and the following illustrative examples, make and utilize the present invention. The following working examples therefore, specifically point out the preferred embodiments of the present invention, and are not to be construed as limiting in any way the remainder of the disclosure.

### Examples

### 1.1 Clinical Study Design

A purpose of this study is to determine the pharmacokinetic and pharmacodynamic interaction of warfarin and trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate (Compound L). A significant number of heart failure or hypertension patients are expected to be on warfarin treatment with other co-medication. When this study was conducted, no information was available on the effect of trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate (Compound L) on the pharmacokinetic and pharmacodynamic profiles of warfarin. Since it was identified that (2R,4S)-5-biphenyl-4-yl-4(3-carboxy-propionyl amino)-2-methyl-pentanoic acid is a weak inhibitor of CYP 2C9, a major metabolizing enzyme of (S)-warfarin, the results from the proposed study would be helpful in determining whether compound L and warfarin can be co-administered safely to patients.

### 1.2 Study design rationale

This study employs a single blind, randomized, two-period, crossover design. All subjects are blinded to the treatment to eliminate study bias. Each subject participates in a screening period, two baseline periods, and two treatment periods. A washout period of at least 10 days but not more than 14 days separates each treatment period.

Due to high variability associated with pharmacokinetics and pharmacodynamics of warfarin a cross over design is proposed to reduce the inter-subject variability. Since warfarin is eliminated with a terminal half-life of approximately 40 hrs, a washout period of at least 10 days is proposed between treatment phases.

### 1.3 Study design overview

This study employs a single blind, randomized, two-period, crossover design. Twenty six healthy male and female subjects are enrolled to ensure at least 20 completers. Each subject participates in a screening period (day -21 to day -2), two baseline periods (day -1), two treatment periods, a washout period of at least 10 days separating the treatment periods, and a study completion evaluation. Subjects undergo routine safety testing during screening, including physical, routine hematology, biochemistry, urinalysis, viral serology screening, pregnancy testing (female subjects), urine drug screening, alcohol screening, standard 12-lead electrocardiogram (ECG), and vital signs assessments to establish eligibility.
Subjects are randomized into the following two treatment sequences during treatment Period 1 and Period 2 as shown.

**Table 1: Study design**

| | | | |
|---|---|---|---|
| Sequence | Period 1 | Washout period (minimum of 10 days) | Period 2 |
| I | 200 mg Compound L b.i.d for 10 days single dose of warfarin sodium 25 mg is administered on Day 5 along with morning Compound L dose | | Placebo tablet b.i.d for 10 days. A single dose of warfarin sodium 25 mg is administered on Day 5 along with morning placebo dose |
| II | Placebo tablet b.i.d for 10 days. A single dose of warfarin sodium 25 mg is administered on Day 5 along with morning placebo dose | | 200 mg Compound L b.i.d for 10 days single dose of warfarin sodium 25 mg is administered on Day 5 along with morning Compound L dose |

Treatment Period 1: Those subjects who successfully pass screening (Days -21 to -2) report to the study center in the morning of the day prior to the initial dosing (Day -1) for admission into the study center, at which time they undergo baseline safety evaluations as conducted during screening (except viral serology). All baseline safety evaluation results should be available prior to first dosing. Subjects are domiciled for at least 11 days during each treatment period. Prothrombin assessments are done at multiple time-points (post-warfarin dose) throughout each period.

On Day 1, subjects are administered the study drug, 200 mg trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate b.i.d. or its matching placebo depending on the treatment sequence he or she is randomized to and continue to receive the same for up to 10 days. On Day 5, a single dose of 25 mg warfarin is co-administered along with trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate or placebo morning dose.

Pre-dose pharmacokinetic samples for trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate are collected on Day 3 [both morning and evening drug/placebo dosing] and on Day 4 (morning only). Serial samples for drug/placebo pharmacokinetics are collected on Day 4 for up to 12 hours post morning dose. On Day 5, serial pharmacokinetic samples are obtained for up to 144 hours for warfarin (both drug/ placebo treatments and warfarin) and for up to 12 hours for drug/placebo.

In the evening prior to each of full pharmacokinetic (PK) assessment days (Day 4 & day 5) the subjects fast overnight (10-12 hours) prior to dosing, and continue to fast until 4 hours post-dose. On Day 1, 2, 3, 6, 7, 8, 9, and 10 the subjects are dosed following an overnight fast of 10-12 hours and receive breakfast 30 minutes post-dose, and evening snacks are provided 30 minutes after evening dosing on all dosing days.

Treatment Period 2: Subjects are required to return to the study center following a washout period of at least 10 days for period 2 baseline evaluation (Day -1). All study related activities including pharmacokinetic sampling for all treatments follow the same schedule as in Period 1.

**Table 2: Pharmacokinetic (PK) Results**

| Analyte | Parameter | Treatment | N | Adjusted Geometric means | Ratio (± LCZ696) | 90% CI for ratio |
|---|---|---|---|---|---|---|
| R-Warfarin | AUCinf [hr*ng/mL] | LCZ696 200 mg + Warfarin 25 mg | 25 | 64891.96 | 0.98 | 0.96, 1.00 |
| | | Placebo + Warfarin 25 mg | 25 | 66153.48 | | |
| | AUClast [hr*ng/mL] | LCZ696 200 mg + Warfarin 25 mg | 25 | 58796.87 | 0.99 | 0.97, 1.01 |
| | | Placebo + Warfarin 25 mg | 25 | 59534.11 | | |
| | Cmax [ng/mL] | LCZ696 200 mg + Warfarin 25 mg | 25 | 1325.29 | 0.96 | 0.91, 1.03 |
| | | Placebo + Warfarin 25 mg | 25 | 1373.71 | | |
| S-Warfarin | AUCinf [hr*ng/mL] | LCZ696 200 mg + Warfarin 25 mg | 25 | 50677.20 | 0.97 | 0.95, 1.00 |
| | | Placebo + Warfarin 25 mg | 25 | 52027.31 | | |
| | AUClast [hr*ng/mL] | LCZ696 200 mg + Warfarin 25 mg | 25 | 46647.81 | 0.98 | 0.95, 1.01 |
| | | Placebo + Warfarin 25 mg | 25 | 47570.37 | | |
| | Cmax [ng/mL] | LCZ696 200 mg + Warfarin 25 mg | 25 | 1344.45 | 0.95 | 0.88, 1.03 |
| | | Placebo + Warfarin 25 mg | 25 | 1414.20 | | |

AUCinf refers to Area Under the Curve infinity in [hr ng/mL] indicating the integrated quantity of analyte or drug (the serum concentration curve) after dosing.

AUClast refers to Area Under the Curve last sample in [hr ng/mL] where activity could be detected.

Cmax refers to the maximum concentration of the analyte or drug in [ng/mL] achieved after dosing.

**Table 3: Pharmacodynamic (PD) Results**

| | | | | Ratio of Geometric mean (± LCZ696) | |
|---|---|---|---|---|---|
| Variable | Treatment | N | Geometric means | Point estimate | 90% CI |
| Mean Prothrombin time (AUCPT/144) (sec) | LCZ696 200 mg + Warfarin 25 mg | 25 | 16.45 | 1.00 | 0.99, 1.01 |
| | Placebo + Warfarin 25 mg | 25 | 16.51 | | |
| Peak prothrombin time* (sec) | LCZ696 200 mg + Warfarin 25 mg | 25 | 21.88 | 0.99 | 0.97, 1.02 |
| | Placebo + Warfarin 25 mg | 25 | 22.00 | | |
| Mean INR(AUCINR/144) | LCZ696 200 mg + Warfarin 25 mg | 25 | 1.29 | 1.00 | 0.98, 1.01 |
| | Placebo + Warfarin 25 mg | 25 | 1.30 | | |
| Peak INR^{§} | LCZ696 200 mg + Warfarin 25 mg | 25 | 1.88 | 0.99 | 0.95, 1.03 |
| | Placebo + Warfarin 25 mg | 25 | 1.90 | | |

| | | | | | |
|---|---|---|---|---|---|
| * Median time to peak PT: 36 hours for LCZ696 + Warfarin; 36 hours for placebo + Warfarin ^{§} Median time to peak INR: 36 hours for LCZ696 + Warfarin; 36 hours for placebo + Warfarin | | | | | |

### Results

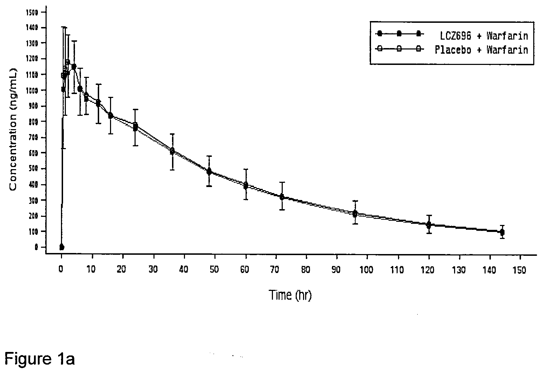

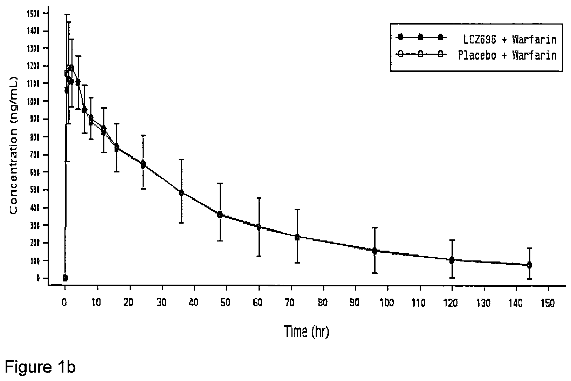

The results from the pharmacokinetic (PK) analysis in Table 2 indicate that the 90% confidence intervals (CI) of the geometric mean ratio for both AUC and Cmax of R- and S-warfarin, with LCZ696 and without LCZ696 (placebo) were within 80 - 125% range (Figures 1a and 1b). Hence, no steady-state drug interaction was found when LCZ696 200 mg b.i.d and warfarin 25 mg single dose were co-administered in a human subject. Co-administration of LCZ696 and warfarin did not change the pharmacokinetics of LCZ696.

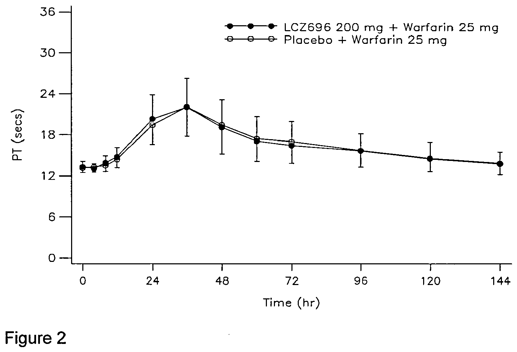

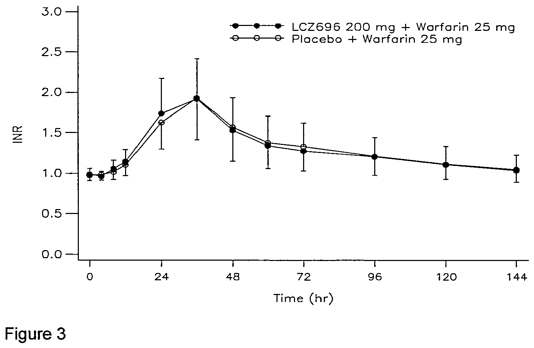

The results from the pharmacodynamic (PD) analysis in Table 3 indicate that LCZ696 200 mg b.i.d. did not affect or impact the anticoagulant properties of warfarin as reflected in the prothrombin time and INR time-course following intake of warfarin 25 mg single dose (Figures 2 and 3).

In summary, it was found that LCZ696 (compound L) could be co-administered safely to a person receiving warfarin treatment and not require further adjustment of the warfarin dosage due to the absence of interaction with LCZ696 and warfarin.

## Claims

1. A pharmaceutical composition for use in the prevention or treatment of heart failure in a human receiving anti-coagulant therapy comprising:
a therapeutically effective amount of the compound trisodium [3-((1*S*,3*R*)-1-biphenyl-4-ylmethyl-3-ethoxycarbonyl-1-butylcarbamoyl)propionate-(*S*)-3'-methyl-2'-(pentanoyl{2"-(tetrazol-5-ylate)biphenyl-4'-ylmethyl}amino)butyrate] hemipentahydrate
in combination with one or more pharmaceutically acceptable carriers,
wherein the anticoagulant treatment comprises administration of warfarin or a pharmaceutically acceptable salt thereof,
wherein the co-administration of the pharmaceutical composition and warfarin did not show pharmacokinetic drug-drug interaction.

2. The pharmaceutical composition for use according to claim 1 wherein the heart failure is congestive heart failure, left heart failure, right heart failure, chronic heart failure, advanced heart failure, acute heart failure, acute decompensated heart failure, heart failure with reduced ejection fraction, or heart failure with preserved ejection fraction.

3. The pharmaceutical composition for use according to any one of the preceding claims wherein the human has previously suffered a myocardial infarction.

4. The pharmaceutical composition for use according to any one of the preceding claims wherein the human has an enlarged heart.

5. The pharmaceutical composition for use according to any one of the preceding claims wherein the human has atherosclerosis.

## Patentansprüche

1. Pharmazeutische Zusammensetzung zur Verwendung bei der Prävention oder Behandlung von Herzinsuffizienz bei einem eine Antikoagulationstherapie erhaltenden Menschen, umfassend:
eine therapeutisch wirksame Menge der Verbindung Trinatrium-[3-((1*S*,3*R*)-1-biphenyl-4-ylmethyl-3-ethoxycarbonyl-1-butylcarbamoy)-propionat-(*S*)-3'-methyl-2'-(pentanoyl{2"-(tetrazol-5-ylat)biphenyl-4'-ylmethyl}amino)butyrat]-hemipentahydrat
in Kombination mit einem oder mehreren pharmazeutisch unbedenklichen Trägern,
wobei die Antikoagulationsbehandlung die Verabreichung von Warfarin oder eines pharmazeutisch unbedenklichen Salzes davon umfasst,
wobei die gemeinsame Verabreichung der pharmazeutischen Zusammensetzung mit Warfarin keine pharmakokinetische Arzneimittelwechselwirkung zeigte.

2. Pharmazeutische Zusammensetzung zur Verwendung nach Anspruch 1, wobei es sich bei der Herzinsuffizienz um Stauung sinsuffizienz, Linksherzinsuffizienz, Rechtsherzinsuffizienz, chronische Herzinsuffizienz, fortgeschrittene Herzinsuffizienz, akute Herzinsuffizienz, akute dekompensierte Herzinsuffizienz, Herzinsuffizienz mit verminderter Ejektionsfraktion oder Herzinsuffizienz mit konservierter Ejektionsfraktion handelt.

3. Pharmazeutische Zusammensetzung zur Verwendung nach einem der vorhergehenden Ansprüche, wobei der Mensch bereits einen Herzinfarkt erlitten hat.

4. Pharmazeutische Zusammensetzung zur Verwendung nach einem der vorhergehenden Ansprüche, wobei der Mensch ein vergrößertes Herz hat.

5. Pharmazeutische Zusammensetzung zur Verwendung nach einem der vorhergehenden Ansprüche, wobei der Mensch an Atherosklerose leidet.

## Revendications

1. Composition pharmaceutique pour utilisation dans la prévention ou le traitement de l'insuffisance cardiaque chez un humain recevant une thérapie anticoagulante comprenant :
une quantité thérapeutiquement efficace du composé hémipentahydrate de [3-((1S,3R)-1-biphényl-4-ylméthyl-3-éthoxycarbonyl-1-butylcarbamoyl)propionate-(S)-3'-méthyl-2'-(pentanoyl{2"-(tétrazol-5-ylate)biphényl-4'-ylméthyl} amino)butyrate] trisodique
en combinaison avec un ou plusieurs véhicules pharmaceutiquement acceptables,
le traitement anticoagulant comprenant l'administration de warfarine ou un sel pharmaceutiquement acceptable de celle-ci,
la co-administration de la composition pharmaceutique et de warfarine ne présentant pas d'interaction médicament-médicament pharmacocinétique.

2. Composition pharmaceutique pour utilisation selon la revendication 1, l'insuffisance cardiaque étant une insuffisance cardiaque congestive, une insuffisance cardiaque gauche, une insuffisance cardiaque droite, une insuffisance cardiaque chronique, une insuffisance cardiaque avancée, une insuffisance cardiaque aiguë, une insuffisance cardiaque aiguë décompensée, une insuffisance cardiaque avec fraction d'éjection réduite ou une insuffisance cardiaque avec fraction d'éjection préservée.

3. Composition pharmaceutique pour utilisation selon l'une quelconque des revendications précédentes, l'humain ayant précédemment souffert d'un infarctus du myocarde.

4. Composition pharmaceutique pour utilisation selon l'une quelconque des revendications précédentes, l'humain ayant un coeur hypertrophié.

5. Composition pharmaceutique pour utilisation selon l'une quelconque des revendications précédentes, l'humain étant atteint d'athérosclérose.
